# EUROPÄISCHE PATENTSCHRIFT

(11) **EP 1 639 958 B1**
(45) Veröffentlichungstag und Bekanntmachung des Hinweises auf die Patenterteilung: **25.07.2007**
(21) Anmeldenummer: 04022937.9
(22) Anmeldetag: 27.09.2004
(51) Int. Cl.: A61B 19/00, G02B 5/12, G02B 5/128

(54) **Rückstrahlender Marker und Verfahren zu seiner Herstellung**
Retroreflective marker and manufacturing method thereof
Cible rétro-réfléchissante et son procédé de fabrication

(43) Veröffentlichungstag der Anmeldung: 29.03.2006
(73) Patentinhaber: BrainLAB AG, 85551 Kirchheim/Heimstetten (DE)
(72) Erfinder: Rossner, Holger-Claus, 85622 Feldkirchen (DE); Pittroff, Thomas, 85570 Markt Schwaben (DE)
(74) Vertreter: Schwabe - Sandmair - Marx

(56) Entgegenhaltungen:
- WO-A-20/04075768
- FR-A- 2 706 045
- GB-A- 1 531 480
- US-A- 2 440 584
- US-A- 3 175 935
- US-A- 3 254 563
- US-A1- 2002 068 942
- US-B1- 6 351 659

## Beschreibung

Die vorliegende Erfindung bezieht sich auf einen rückstrahlenden Marker, wobei ein solcher Marker insbesondere eine dreidimensionale Form, wie eine Kugelform, aufweisen und im medizinischen Bereich, zum Beispiel bei einer Operation, als eine zum Beispiel an dem Körper des Patienten angebrachte Hilfe zur Registrierung und Navigation des Körpers verwendet werden können. Die rückstrahlenden oder retro-reflexiven Eigenschaften resultieren insbesondere aus einer speziellen Oberflächenbeschichtung oder Oberflächenstruktur, welche mittels verschiedener Verfahren, wie Aufspritz-, Streich-, Mal-, Lackier- oder Eintauchverfahren, erhalten werden können.

Um möglichst gute rückstrahlende oder retro-reflexive Eigenschaften aufzuweisen, sollte die Oberfläche der Marker eine gleichmäßige Struktur haben, weshalb allgemein Herstellungsverfahren bevorzugt werden, mit welchen Marker hergestellt werden, die vorzugsweise an jeder Stelle ihrer Oberfläche dieselben rückstrahlenden oder retro-reflexiven Eigenschaften aufweisen.

Die US-3,964,820 beschreibt ein kugelförmiges retro-reflexives Element, welches einen Lichtstrahl entlang seines Einfallsweges rückstrahlt, wenn Licht auf dessen teilkugelförmigen Oberflächenteil auftrifft. Auf der übrigen Oberfläche des Elements ist eine Vielzahl konkaver und reflektierender teilkugelförmiger kleiner Kugeln vorgesehen, welche Lichtstrahlen zurücksenden, die auf den teilkugelförmigen Oberflächenanteil des Elementes aufgetroffen sind und ins Innere des Elementes gebrochen wurden.

Die US-3,971,692 bezieht sich auf ein retro-reflexives Material, welches hergestellt wird, indem eine Schicht transparenter Glaskugeln auf einen klebrigen Überzug aufgebracht wird, der auf einem Trägerblatt ausgebildet ist, wobei die freiliegenden Teile der Kugeln mit einem reflektierendem Material, wie Aluminium, bestrichen werden und die bestrichenen Teile der Kugeln in einen klebenden Belag eines Bauteils eingelassen werden, wobei anschließend die Trägerfolie abgezogen wird.

Die US-4,265,938 betrifft Retro-Reflektoren wie sie für Straßenschilder und Fahrzeugnummernschilder verwendet werden. Dabei wird die Oberfläche eines metallischen Substrats retro-reflexiv gemacht, indem eine Schicht aus einem organischen Polymer und Glaskugeln angeklebt wird und das Substrat anschließend durch eine Walze geleitet wird, wobei die Glaskugeln mit einem Tiegel bedeckt sind, so dass die Kugeln Vertiefungen in der metallischen Oberfläche erzeugen.

Die FR 2 706 045 betrifft retro-reflektierende Scheiben und deren Herstellung, wobei die retro-reflektierenden Scheiben insbesondere für die Inspektion, die Messung, oder die Beobachtung einer Oberfläche eines Objekts verwendet werden. Auf einen metallischen Innenkörper der Scheibe wird ein erster reflektierender Überzug aufgebracht und auf die Oberfläche des ersten reflektierenden Überzugs wird ein zweiter Überzug aufgebracht, wobei in den zweiten Überzug Glaskügelchen eingebracht werden.

Die WO 2004/075768 betrifft justierbare Markeranordnungen, Werkzeuge und Verfahren, wobei ein justierbarer Marker einen abbildbaren sphärischen justierbaren Lokalisierer enthält. Die äußere Oberfläche des sphärischen justierbaren Lokalisierers enthält lichtreflektierende Mikrokugeln, welche in einem Klebstoff eingebettet sind.

Die US 3,254,563 bezieht sich auf retro-reflektierende Teilchen und reflektierende Marker und reflektierende Sphäroide, wobei die reflektierenden Sphäroide im Wesentlichen kugelförmig geformte vollständig ausgehärtete monolithische Kerne eines duroplastischen Bindematerials sind, wobei die gesamte äußere Oberfläche mit Glaskügelchen bedeckt ist, welche teilweise in die Oberfläche eingebettet sind.

Die US 3,175,935 betrifft ein Verfahren zur Herstellung reflektierender Teilchen und zum Überziehen von Innenkörpern oder Körnchen, wobei reflektierende Körnchen gebildet werden, indem ein von einer Bindeschicht umgebener Kern und reflex-reflektierende Komplexe bestehend aus transparenten Mikrokugeln mit unterliegenden Reflektoren verwendet werden. Zum Herstellen der reflektierenden Körnchen werden harzüberzogene Kerne verwendet.

Es ist eine Aufgabe der vorliegenden Erfindung vorzugsweise gleichmaßig rückstrahlende oder retro-reflexive dreidimensionale Körper bzw. Marker für den medizinischen Bereich vorzuschlagen.

Diese Aufgabe wird durch den Gegenstand des unabhängigen Anspruchs gelöst. Vorteilhafte Ausführungsformen ergeben sich aus den abhängigen Ansprüchen.

Bei einem Verfahren zur Herstellung erfindungsgemäßer rückstrahlender oder retro-reflexiver Marker, welche aus einem Innenkörper oder Basiskörper oder 3D-Körper und einem darauf aufgebrachten rückstrahlenden Überzug bestehen, wird vorzugsweise unmittelbar auf den Innenkörper, welcher bevorzugt aus einem transparenten oder nicht transparenten Material, insbesondere aus Plastik, hergestellt wird, eine flüssige reflektierende Substanz oder Trägersubstanz aufgebracht. Dabei kann die flüssige reflektierende Substanz ein körniges oder granuliertes Material, wie Granulat, Kügelchen, vorzugsweise aus transparentem Material, insbesondere aus Glas, und ein reflektierendes Material, insbesondere Aluminiumpulver, Silberpulver oder Goldpulver, enthalten. Insbesondere können auch das Granulat und/oder die Kügelchen und/oder das reflektierende Material in einer flüssigen Substanz, wie einem Lack, enthalten sein oder mit dieser vermischt werden oder sein.

Dabei wird unter einer flüssigen Substanz im Sinne oder Erfindung nicht zwangsläufig eine Substanz im flüssigen Aggregatzustand verstanden. Erfindungsgemäß kann eine flüssige Substanz in flüssiger Phase vorliegen, sie kann jedoch auch eine Mischung von Substanzen oder eine Mischung von Phasen oder eine Dispersion sein, wobei sich in dem Gemenge der mindestens zwei Substanzen oder Stoffe die Stoffe ineinander lösen können oder sich ineinander nicht oder kaum lösen können oder kaum chemisch miteinander verbinden können. Beispielsweise kann in einer festen oder flüssigen oder gasförmigen Phase oder Substanz, zum Beispiel als Dispersionsmittel, eine feste und/oder flüssige und/oder gasförmige Phase oder Substanz, zum Beispiel als disperse Phase, verteilt oder enthalten oder gelöst sein, wobei bevorzugt in einer flüssigen oder gasförmigen Phase eine feste und/oder flüssige und/oder gasförmige Phase enthalten oder verteilt sein kann, so dass zum Beispiel gelartige oder schaumartige Substanzen sowie Suspensionen oder Emulsionen oder Lösungen erfindungsgemäß als flüssige Substanz verstanden werden können. Insbesondere werden alle Substanzen erfindungsgemäß als flüssige Substanz bezeichnet, die dazu geeignet sind mit oder ohne den Kügelchen auf den Innenkörper aufgebracht zu werden, so dass die in der Substanz enthaltenen oder nach dem Aufbringen der Substanz aufgebrachten Kügelchen vorzugsweise in etwa zu 50% in die Substanz eintauchen können oder in der Substanz eingelassen sind oder in die Substanz eingelassen werden und mit der Substanz einen rückstrahlenden, insbesondere retro-reflexiven Überzug bilden,

Der verwendete Innenkörper oder 3D-Körper kann vollständig ausgefüllt, zum Beispiel massiv, sein, er kann jedoch auch mit einem Hohlraum erzeugt werden oder es kann nach der Herstellung des Innenkörpers ein Hohlraum in dem Innenkörper erzeugt werden. Des Weiteren kann an dem Innenkörper eine Befestigungsvorrichtung, insbesondere ein Loch, eine Einkerbung oder ein Stab, vorgesehen sein, mit welcher der Marker an einem Träger befestigt werden kann, um so mit anderen an dem Träger angebrachten Markern ein Markerarray, wie einen Referenzstern, bilden zu können. Bevorzugt wird die an dem Innenkörper angebrachte Befestigungsvorrichtung während des Herstellungsvorganges geschützt, um ein späteres sicheres Anbringen des fertiggestellten Markers zu gewährleisten.

Die flüssige reflektierende Substanz kann vor dem Aufbringen auf den Innenkörper mit oder aus einem körnigen oder granulierten Material, insbesondere Granulat, wie Kunststoffkörner oder -komponenten und/oder Kügelchen und/oder reflektierendem Material, wie reflektierendem Pulver, zum Beispiel Aluminium-, Silber- oder Goldpulver, gemischt oder angemischt werden. Die flüssige reflektierende Substanz kann zum Beispiel gebildet werden, indem eine flüssige Substanz, wie ein Lack, mit dem Granulat und/oder den Kügelchen und/oder dem reflektierenden Material oder Pulver gemischt oder vermengt wird. Auch kann die flüssige reflektierende Substanz ein reflektierender Klebstoff sein. Insbesondere weist die flüssige reflektierende Substanz ihre reflektierenden Eigenschaften in einem zumindest teilweise, insbesondere völlig getrockneten Zustand auf und muss nicht zwangsläufig schon im flüssigen Zustand reflektierende Eigenschafen besitzen, unabhängig davon mit oder aus weicher der genannten Komponenten die flüssige reflektierende Substanz oder Trägersubstanz gemischt oder hergestellt wird.

Insbesondere kann die flüssige reflektierende Substanz gleichmäßig auf dem Innenkörper aufgebracht sein, so dass die Substanz im getrockneten Zustand radial einen rückstrahlenden oder retro-reflexiven Überzug oder Außenkörper konstanter bzw. gleichmäßiger Dicke um den Innenkörper ausbildet.

Mit dem beschriebenen Verfahren können Marker hergestellt werden, welche an jeder Stelle in etwa dieselben rückstrahlenden oder retro-reflexiven Eigenschaften aufweisen. Die bekannten Verfahren sind entweder nicht zur Herstellung dreidimensionaler Marker einsetzbar oder es kann keine gleichmäßig rückstrahlende oder retro-reflexive Oberfläche aufgebracht werden, da es, wie zum Beispiel bei dem Aufbringen einer reflektierenden Folie, zu Unstetigkeiten wie Nahtstellen kommen kann.

Vorzugsweise wird außerdem eine Sterilisierung der Marker, insbesondere mit Ethylengas, durchgeführt, um desinfizierte, keimfreie und sterile Marker mit zum Beispiel maximal 10 Keimen pro Marker zu erhalten.

Vorteilhaft sind die Marker oder Bestandteile davon, also zum Beispiel die verwendeten reflektierenden Materialen, so ausgelegt, dass die reflektierende Eigenschaft durch ein weiteres Sterilisationsverfahren, wie zum Beispiel eine Sterilisation mit Dampf, verloren gehen, wodurch sichergestellt werden kann, dass die Marker in einem absolut sterilen Zustand verwendet werden. Dies kann als eine Art Sterilitäts- und/oder Qualitätssicherungsverfahren betrachtet werden. Würden die rückstrahlenden oder retro-reflexiven Eigenschaften nicht verloren gehen, so könnten die Marker beliebig oft zum Beispiel bei einer Operation eingesetzt werden, so dass die hohen Anforderungen an die Sterilität der Marker zwangsläufig nicht eingehalten werden können. Verlieren die Marker ihre rückstrahlenden oder retro-reflexiven Eigenschaften nach einer erneuten Sterilisation, so sind sie zum Beispiel für die Registrierung und/oder Navigation eines Körpers nicht mehr einsetzbar. Dies gewährleistet, dass stets neue, zum Beispiel vom Hersteller auf Keimfreiheit geprüfte, Marker verwendet werden, die den hohen Sterilitätsanforderungen genügen.

Die erfindungsgemäßen Marker umfassen einen Innenkörper oder Basiskörper oder 3D-Körper und einen auf einer Außenoberfläche des Innenkörpers vorzugsweise unmittelbar auf dem Innenkörper aufgebrachten äußeren Überzug oder Außenkörper, bestehend aus einer reflektierenden Substanz, welche auch adhäsive Eigenschaften aufweisen kann, mit darin zumindest teilweise, bevorzugt in etwa zur Hälfte, eingelassenen Kügelchen, wobei die Kügelchen vorzugsweise aus einem transparenten Material, insbesondere aus Glas, bestehen. Diese Anordnung führt zu gleichmäßigen rückstrahlenden oder retro-reflexiven Eigenschaften des Markers, resultierend aus dem Brechungsindex der Kügelchen in Kombination mit den reflektierenden Eigenschaften der Substanz bzw. des Materials, in das die Kügelchen zumindest teilweise eingelassen sind. Auf dem Marker einfallende Lichtstrahlen können an der Oberfläche der Kügelchen gebrochen werden, treffen nach Durchdringen der vorzugsweise transparenten Kügelchen auf die Oberfläche des reflektierenden Überzugs, werden an diesem reflektiert und können nach erneutem Durchdringen der Kügelchen so an der Oberfläche der Kügelchen gebrochen werden, dass der Lichtstrahl in die ähnliche Richtung abgestrahlt wird aus der er eingefallen ist. Folglich kann der Lichtstrahl zumindest nahezu an seinen Sender zurückgestrahlt werden, womit der Marker rückstrahlende oder retro-reflexive Eigenschaften besitzt.

Auch kann der rückstrahlende Marker einen Innenkörper und eine Klebeschicht umfassen, die vorzugsweise gleichmäßig um den Innenkörper aufgebracht ist, und in die Klebeschicht eingebrachte Kügelchen umfassen, die zumindest zum Teil mit einer reflektierenden Substanz beschichtet sind, wobei vorzugsweise der zu dem Innenkörper weisende Teil der Kügelchenoberfläche mit der reflektierenden Substanz beschichtet ist und der von dem Innenkörper weg weisende oder nach außen weisende Teil der Kügelchenaußenoberfläche frei von der reflektierenden Substanz ist.

Insbesondere besteht der Innenkörper des rückstrahlenden Markers aus einem transparenten oder nicht transparenten Material, wie aus Plastik, so dass einfallende Lichtstrahlen, die zum Beispiel an der Oberfläche der Kügelchen gebrochen werden und auf die reflektierende Substanz oder den Innenkörper treffen können, nicht ins Innere des Innenkörpers gelangen, sondern an diesem reflektiert oder absorbiert werden. Ebenso kann der Innenkörper des Markers auch aus der reflektierenden Substanz selbst bestehen, zum Beispiel aus dem körnigen oder granulierten Material, wie Granulat, und aus den stark reflektierenden Komponenten, wie Aluminium-, Silber- oder Goldpulver, so dass der gesamte Marker vollständig aus dieser Substanz gebildet werden kann und zum Beispiel in einem teilweise getrockneten Zustand der Substanz Kügelchen auf der Außenoberfläche angebracht werden können. Der Innenkörper kann auch einen Hohlraum aufweisen und es kann an ihm eine Befestigungsvorrichtung, insbesondere ein Loch, eine Einkerbung oder ein Stab, angebracht sein, mit der der Marker an einem Halteelement, insbesondere an einem Referenzstern, befestigt werden kann.

Die zur Ausbildung des äußeren Überzugs oder des Innenkörpers verwendete reflektierende Substanz enthält vorzugsweise ein körniges oder granuliertes Material und/oder Kügelchen, vorzugsweise aus einem transparenten Material, wie z.B. Glas, und/oder ein reflektierendes Material, wie Aluminium-, Silber- oder Goldpulver. Diese Substanz ist bevorzugt so auf dem Innenkörper aufgebracht, dass sie um den Innenkörper einen äußeren Überzug oder einen Außenkörper bildet, in dem die Kügelchen zumindest teilweise eingelassen sind. Um die bestmöglichen rückstrahlenden Eigenschaften des Markers zu gewährleisten sollten die Kügelchen, die vorzugsweise einen Durchmesser zwischen 10 und 250 µm, insbesondere von 180 µm, haben, zur Hälfte in dem äußeren Überzug eingelassen sein, so dass auf die Kügelchen treffende Lichtstrahlen ins Innere der Kügelchen gebrochen werden und am reflektierenden Überzug rückgestrahlt werden. Bevorzugt sind die Kügelchen unmittelbar nebeneinander angeordnet, so dass auf den Marker gestrahlte Lichtstrahlen vorzugsweise auf die Kügelchen treffen. Der äußere Überzug oder der Außenkörper kann auch gleichmäßig auf den Innenkörper aufgebracht sein und insbesondere einen Überzug oder einen Außenkörper gleichmäßiger Dicke ausbilden, um dasselbe rückstrahlende oder retro-reflexive Verhalten an jeder Stelle des Markers zu gewährleisten.

Der rückstrahlende Marker kann verschiedene Formen besitzen, insbesondere kann er kugelförmig, zylinderförmig, pyramidenförmig oder prismaförmig sein, wobei er, wenn er als Kugel oder kugelförmig ausgebildet ist, bevorzugt einen Durchmesser zwischen 5 und 50 mm, insbesondere von 13,2 mm, hat, wobei Schwankungen von einigen Hundertstel Millimetern, wie etwa 0,04 mm, auftreten können und zum Beispiel weniger als 2 Gramm wiegt.

Die Erfindung wird nachfolgend anhand bevorzugter Ausführungsbeispiele unter Bezugnahme auf die Zeichnungen beschrieben werden. Es zeigen:
- Figur 1: eine erste Ausführungsform eines erfindungsgemäßen rückstrahlenden Markers, wobei der retro-reflexive Überzug aus einer Mischung aus körnigem oder granuliertem Material, Kügelchen und stark reflektierendem Pulver besteht;
- Figur 2: eine weitere Ausführungsform eines erfindungsgemäßen rückstrahlenden Markers, wobei die retro-reflexive Schicht aus einer Mischung aus körnigem oder granuliertem Material, Kügelchen und Klebstoff besteht;
- Figur 3a: eine weitere Ausführungsform eines erfindungsgemäßen rückstrahlenden Markers mit einem reflektierenden Überzug und darin eingelassenen Kügelchen;
- Figur 3b: eine weitere Ausführungsform des erfindungsgemäßen rückstrahlenden Markers mit Kügelchen und einer auf den Kügelchen aufgebrachten reflektierenden Substanz;
- Figur 3c: eine weitere Ausführungsform eines erfindungsgemäßen rückstrahlenden Markers mit einer Klebeschicht und in die Klebeschicht eingebrachten teilweise verspiegelten Kügelchen.

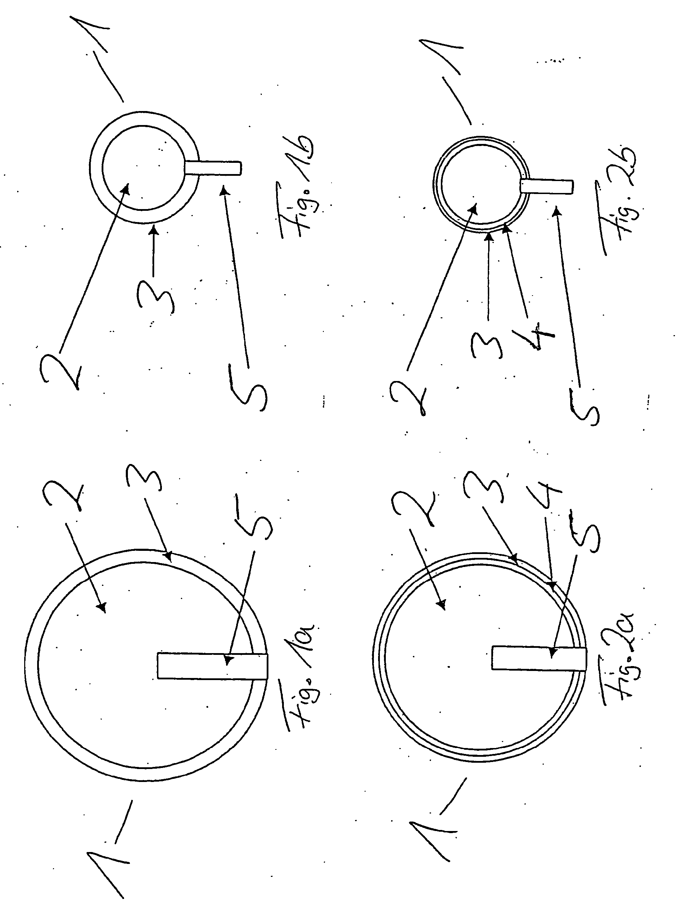

Figur 1a zeigt den Querschnitt eines kugelförmigen rückstrahlenden oder retro-reflexiven Markers 1, dessen Innenkörper 2 aus Plastik oder einem Kunststoff besteht, auf welchem unmittelbar ein Überzug 3 mit rückstrahlenden oder retro-reflexiven Eigenschaften aufgebracht ist. Dieser rückstrahlende Überzug 3 besteht aus einer Mischung aus einem körnigen oder granulierten Material, wie Plastik- oder Kunststoffgranulat, Kügelchen, wie Glaskügelchen, und reflektierendem Pulver, wie Aluminium-, Silber- oder Goldpulver. Zunächst ist der Überzug 3 nicht auf dem Innenkörper 2 aufgebracht, sondern wird in einem flüssigen Zustand aus mindestens einer der Komponenten, dem Granulat, den Kügelchen und dem reflektierenden Pulver, gemischt und auf den Innenkörper 2 aufgespritzt, aufgestrichen, aufgemalt oder auflackiert oder der innenkörper 2 wird in ein aus der flüssigen Substanz bestehendes Immersionsbad eingetaucht, so dass sich im getrockneten Zustand ein bevorzugt gleichmäßiger Überzug 3 auf dem Innenkörper 2 ergibt, der rückstrahlende oder retro-reflexive Eigenschaften aufweist. Dabei können die Kügelchen von Beginn an in der flüssigen Substanz enthalten sein und so auf den Innenkörper 2 aufgebracht werden, dass sie im getrockneten Zustand des Überzugs 3 in etwa zur Hälfte in den Überzug eingelassen sind oder sie können nach dem Aufbringen des Überzugs 3 zum Beispiel in einem teilweise getrockneten Zustand der Schicht bzw. des Überzugs 3 aufgespritzt oder aufgeschossen werden, wie zum Beispiel mittels eines Pulverspritzverfahrens oder Thermobeschichtungsverfahrens. An dem Marker 1 ist ein als Befestigungsvorrichtung 5 dienendes Loch angebracht, das zum Beispiel schon vor dem Aufbringen des reflektierenden Überzugs 3 an dem Innenkörper 2 angebracht sein kann oder angebracht werden kann und während des Aufbringens des Überzugs 3 geschützt bleibt, so dass das Loch von außerhalb des Markers 1 zugänglich ist und zum Beispiel an einem Halteelement, wie einem Referenzstern, angebracht werden kann.

Figur 1b zeigt den rückstrahlenden Marker 1 aus Figur 1a, wobei als Befestigungsvorrichtung 5 ein Stab an dem Innenkörper 2 bzw. an dem Marker 1 vorgesehen ist, der auch während des Aufbringens des reflektierenden Überzugs 3 geschützt werden kann und somit als Befestigungselement 5 dienen kann, um den Marker 1 zum Beispiel an einem Referenzstern anzubringen.

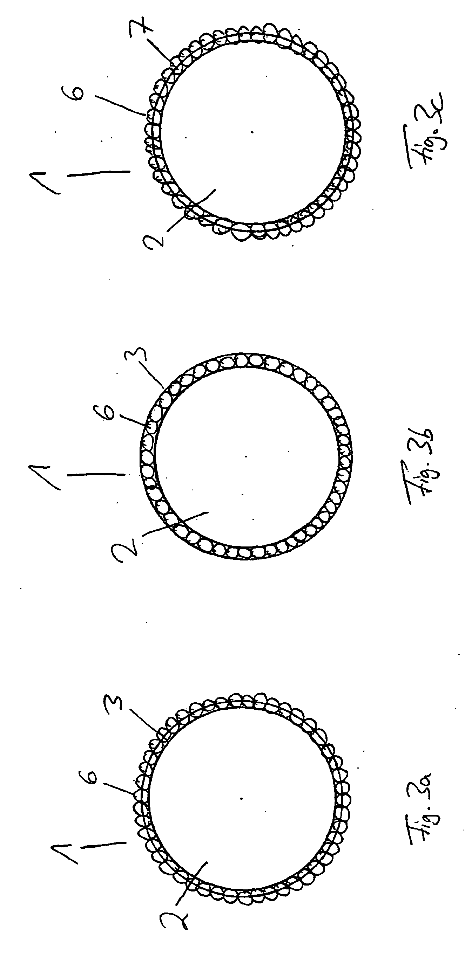

Figur 2a zeigt den Querschnitt eines kugelförmigen rückstrahlenden oder retro-reflexiven Markers 1, wobei auf den Innenkörper 2 ein äußerer Überzug 3 oder ein Außenkörper, bevorzugt bestehend aus körnigem oder gekörntem Material, wie Plastik- oder Kunststoffgranulat, Kügelchen und einem Klebstoff, der reflektierend oder nicht reflektierend sein kann, aufgebracht ist.

Dabei kann der rückstrahlende Überzug 3 auf den Innenkörper 2 aufgebracht werden, indem der Innenkörper 2 elektrostatisch geladen wird und mit einem flüssigen Klebstoff 4 bestrichen oder bedeckt wird, der vorzugsweise in einem getrockneten Zustand reflektierende Eigenschaften besitzt. Anschließend können die Kügelchen entgegengesetzt zu dem Innenkörper 2 elektrostatisch geladen werden und in die Nähe des Innenkörpers 2 gebracht werden, so dass sie von dem Innenkörper 2 auf Grund elektrostatischer Kräfte angezogen werden und an dem Klebstoff 4, der zum Beispiel teilweise getrocknet sein kann, heften bzw. kleben bleiben, so dass die Kügelchen in einem getrockneten Zustand des Klebstoffs 4 zumindest teilweise, bevorzugt in etwa zur Hälfte, in dem Klebstoff 4 eingelassen sind. Die Kombination der Kügelchen, insbesondere deren Brechungseigenschaften, mit der darunter liegenden reflektierenden Klebstoffoberfläche 4 bewirkt starke rückstrahlende oder retro-reflexive Eigenschaften des Überzugs 3 und damit des Markers 1. Auch hier kann eine Befestigungsvorrichtung 5, wie in Figur 2a ein Loch und in Figur 2b ein Stab, an dem Marker 1 bzw an dem Innenkörper 2 angebracht sein oder angebracht werden, die während des Aufbringens des Klebstoffes 4 und/oder der Kügelchen und/oder des Granulats geschützt wird, so dass der Marker 1 an einem Halteelement, wie einem Referenzstern, befestigt werden kann.

Auch kann ein nichtreflektierender Klebstoff 4 auf den Innenkörper 2 aufgebraucht werden, wobei die Kügelchen mit der flüssigen reflektierenden Substanz vollständig oder zumindest teilweise beschichtet werden können und die beschichteten Teile der Kügelchen in den Klebstoff 4 oder die Klebeschicht eingebracht werden. Die zumindest teilweise mit der flüssigen reflektierenden Substanz beschichteten Kügelchen können mit einem der beschriebenen Verfahren in den Klebstoff 4, in etwa zur Hälfte eingebracht werden, so dass der auf dem Klebstoff 4 aufgebrachte oder an dem Klebstoff 4 haftende Überzug 3 aus zumindest teilweise beschichteten Kügelchen rückstrahlende, insbesondere retro-reflexive, Eigenschaften aufweist. Anschließend kann überschüssige Substanz, wie vom Innenkörper weg zeigende oder nicht mit dem Klebstoff 4 verbundene oder an dem Klebstoff 4 haftende Substanz, von den Kügelchen bzw. Kügelchenoberfläche, zum Beispiel durch Ätzen entfernt werden.

Figur 3a zeigt eine weitere Ausführungsform eines erfindungsgemäßen rückstrahlenden Markers 1 mit einem rückstrahlenden Überzug 3, in dem zumindest teilweise, bevorzugt in etwa zu 50%, Kügelchen eingetaucht oder eingebettet sind. Auf dem rückstrahlenden Marker 1 auftreffende Lichtstrahlen werden an der Oberfläche der Kügelchen 6 gebrochen, an dem rückstrahlenden Überzug 3 reflektiert und wiederum an den Kügelchen 6 gebrochen so dass der ausfallende Lichtstrahl zumindest nahezu in dieselbe Richtung rückgestrahlt wird aus der der einfallende Lichtstrahl auf den rückstrahlenden Marker 1 eingefallen ist. Die flüssige reflektierende Substanz wird zur Herstellung des rückstrahlenden Markers 1 vorzugsweise unmittelbar auf den Innenkörper 2 aufgebracht und die Kügelchen 6 werden anschließend auf die flüssige reflektierende Substanz vorzugsweise in einem flüssigen oder teilweise getrockneten Zustand der reflektierenden Substanz, aufgespritzt oder aufgeschossen, so dass die Kügelchen 6 mit der flüssigen reflektierenden Substanz einen rückstrahlenden Überzug 3 bilden und zumindest teilweise, vorteilhaft in etwa zur Hälfte, in den rückstrahlenden Überzug 3 eingebettet werden oder sind.

Figur 3b zeigt eine weitere Ausführungsform eines erfindungsgemäßen rückstrahlenden Markers 1 mit einem Innenkörper 2, wobei zur Herstellung des rückstrahlenden Markers 1 auf den Innenkörper 2 eine reflektierende Substanz aufgespritzt, aufgestrichen, aufgemalt oder auflackiert wird oder der Innenkörper 2 in die reflektierende Substanz eingetaucht wird. Die reflektierende Substanz kann Kügelchen 6 enthalten, so dass die reflektierende Substanz mit den Kügelchen 6 zum Beispiel in einem getrockneten Zustand einen Überzug 3 bildet. Die Kügelchen 6 können zum Beispiel in einem zumindest teilweise getrockneten Zustand der reflektierenden Substanz mit der Substanz beschichtet sein oder von der Substanz umgeben sein. Zumindest ein Teil der Kügelchen 6, vorzugsweise der von dem Innenkörper 2 weg weisende Teil der Kügelchenoberfläche kann von der Substanz befreit werden, indem zum Beispiel die Substanz weggeätzt wird oder indem die Substanz durch einen Trocknungsprozess reduziert oder entfernt wird.

Figur 3c zeigt eine weitere Ausführungsform eines rückstrahlenden Markers 1 mit einem Innenkörper 2, wobei zur Herstellung des rückstrahlenden Markers 1 auf den Innenkörper 2 ein Klebstoff, der reflektierend oder nicht reflektierend sein kann aufgebracht wird, der in einem zumindest teilweise getrockneten Zustand eine vorzugsweise gleichmäßige Klebeschicht 7 um den Innenkörper 2 bildet. Die Kügelchen 6 werden vorzugsweise mit einer reflektierenden Substanz zumindest teilweise verspiegelt oder beschichtet und in die Klebeschicht 7 eingebracht, so dass bevorzugt in etwa 50% jedes Kügelchens 6 in die Klebeschicht 7 eingebettet ist und in etwa 50% jedes Kügelchens 6 freistehend ist oder frei liegt. Im Anschluss können die Kügelchen 6 von der Verspiegelung zum Beispiel mittels eines Ätzvorganges oder eines Waschvorganges befreit werden, wobei vorteilhaft der freistehende oder freiliegende Teil der Kügelchen 6 von der Verspiegelung befreit wird.

## Patentansprüche

1. Steriler rückstrahlender Marker (1) für den medizinischen Bereich zur Navigation und Registrierung, mit einem Innenkörper (2) aus Kunststoff und einer auf einer Außenoberfläche des Innenkörpers (2) vorgesehenen reflektierenden Substanz mit darin eingelassenen Kügelchen (6), wobei
- die reflektierende Substanz adhäsive Eigenschaften aufweist und ein reflektierendes Material, insbesondere Aluminiumpulver, Silberpulver oder Goldpulver, enthält
- der Marker (1) an jeder Stelle in etwa dieselben rückstrahlenden oder retro-reflexiven Eigenschaften aufweist, und
- der Marker (1) so ausgelegt ist, dass die retro-reflexive Eigenschaft des Markers (1) durch ein weiteres Sterilisationsverfahren mit Dampf verloren geht.

2. Marker (1) nach dem vorhergehenden Anspruch, wobei der Innenkörper (2) des Markers (1) aus der reflektierenden Substanz besteht.

3. Marker (1) nach einem der vorhergehenden Ansprüche, wobei der Innenkörper (2) einen Hohlraum aufweist.

4. Marker (1) nach einem der vorhergehenden Ansprüche, wobei an dem Innenkörper (2) eine Befestigungsvorrichtung (5), insbesondere ein Loch, eine Einkerbung oder ein Stab angebracht ist, mit welcher der Marker (1) an einem Halteelement, insbesondere einem Referenzstern, befestigt werden kann.

5. Marker (1) nach einem der vorhergehenden Ansprüche, wobei die reflektierende Substanz weiter ein granuliertes Material enthält.

6. Marker (1) nach einem der vorhergehenden Ansprüche, wobei die reflektierende Substanz so auf dem Innenkörper (2) aufgebracht ist, dass sie um den Innenkörper (2) einen äußeren Überzug (3) bildet, in welchem die Kügelchen (6) zumindest teilweise, insbesondere in etwa zur Hälfte, eingelassen sind.

7. Marker (1) nach dem vorhergehenden Anspruch, wobei der Überzug (3) gleichmäßig auf dem Innenkörper (2) aufgebracht ist, insbesondere einen Überzug (3) gleichmäßiger Dicke bildet.

8. Marker (1) nach einem der vorhergehenden Ansprüche, wobei die Kügelchen (6) aus einem transparentem Material, insbesondere aus Glas, bestehen.

9. Marker (1) nach einem der vorhergehenden Ansprüche, wobei die Kügelchen (6) einen Durchmesser zwischen 10 und 250 µm haben.

10. Marker (1) nach einem der vorhergehenden Ansprüche, wobei die Kügelchen (6) zumindest zum Teil mit der reflektierenden Substanz beschichtet sind.

11. Marker (1) nach einem der vorhergehenden Ansprüche, wobei der Marker (1) kugelförmig, zylinderförmig oder pyramidenförmig ist oder die Form eines Prismas hat.

12. Marker (1) nach einem der vorhergehenden Ansprüche, wobei der Marker (1) einen Durchmesser zwischen 5 und 50 mm hat.

## Claims

1. A sterile reflective marker (1) for the field of medicine for navigating and registering, comprising an inner body (2) made of plastic and a reflective substance provided on an outer surface of the inner body (2) and comprising pellets (6) embedded in it, wherein:
- the reflective substance exhibits adhesive properties and contains a reflective material, in particular aluminium powder, silver powder or gold powder;
- the marker (1) exhibits about the same reflective or retro-reflexive properties at every point; and
- the marker (1) is configured such that the retro-reflexive property of the marker (1) is lost through a further sterilisation method using steam.

2. The marker (1) according to the preceding claim, wherein the inner body (2) of the marker (1) consists of the reflective substance.

3. The marker (1) according to any one of the preceding claims, wherein the inner body (2) exhibits a cavity.

4. The marker (1) according to any one of the preceding claims, wherein a fixing device (5) is attached to the inner body (2), in particular a hole, notch or rod, using which the marker (1) can be fixed to a holding element, in particular a reference star.

5. The marker (1) according to any one of the preceding claims, wherein the reflective substance further contains a granulated material.

6. The marker (1) according to any one of the preceding claims, wherein the reflective substance is applied to the inner body (2) such that it forms an outer covering (3) around the inner body (2), in which the pellets (6) are embedded at least partially, in particular about half-way.

7. The marker (1) according to the preceding claim, wherein the covering (3) is uniformly applied to the inner body (2), in particular forming a covering (3) having a uniform thickness.

8. The marker (1) according to any one of the preceding claims, wherein the pellets (6) consist of a transparent material, in particular glass.

9. The marker (1) according to any one of the preceding claims, wherein the pellets (6) have a diameter between 10 and 250 µm.

10. The marker (1) according to any one of the preceding claims, wherein the pellets (6) are at least partially coated with the reflective substance.

11. The marker (1) according to any one of the preceding claims, wherein the marker (1) is spherical, cylindrical or pyramidal or has the shape of a prism.

12. The marker (1) according to any one of the preceding claims, wherein the marker (1) has a diameter between 5 and 50 mm.

## Revendications

1. Repère (1) rétroréfléchissant stérile pour le domaine médical, pour la navigation et l'enregistrement, comportant un corps intérieur (2) en matière plastique et une substance réfléchissante prévue sur une surface extérieure du corps intérieur (2) avec des billes (6) encastrées à l'intérieur, dans lequel
- la substance réfléchissante présente des propriétés adhésives et contient un matériau réfléchissant, en particulier de la poudre d'aluminium, de la poudre d'argent ou de la poudre d'or,
- le repère (1) présente en tout point à peu près les mêmes propriétés rétroréfléchissantes ou rétroréflectrices, et
- le repère (1) est conçu de manière que la propriété rétroréflectrice du repère (1) est perdue par un procédé ultérieur de stérilisation à la vapeur.

2. Repère (1) selon la revendication précédente, dans lequel le corps intérieur (2) du repère (1) est constitué de la substance réfléchissante.

3. Repère (1) selon l'une des revendications précédentes, dans lequel le corps intérieur (2) présente une cavité.

4. Repère (1) selon l'une des revendications précédentes, dans lequel sur le corps intérieur (2) est mis en place un dispositif de fixation (5), en particulier un trou, une encoche ou une barrette, par lequel le repère (1) peut être fixé sur un élément de retenue, en particulier une étoile de référence.

5. Repère (1) selon l'une des revendications précédentes, dans lequel la substance réfléchissante contient en outre un matériau en granulés.

6. Repère (1) selon l'une des revendications précédentes, dans lequel la substance réfléchissante est appliquée sur le corps intérieur (2) de manière à former autour du corps intérieur (2) un revêtement (3) extérieur dans lequel les billes (6) sont encastrées au moins en partie, en particulier à peu près à moitié.

7. Repère (1) selon la revendication précédente, dans lequel le revêtement (3) est appliqué uniformément sur le corps intérieur (2), et forme en particulier un revêtement (3) d'épaisseur uniforme.

8. Repère (1) selon l'une des revendications précédentes, dans lequel les billes (6) sont constituées d'un matériau transparent, en particulier de verre.

9. Repère (1) selon l'une des revendications précédentes, dans lequel les billes (6) présentent un diamètre compris entre 10 et 250 µm.

10. Repère (1) selon l'une des revendications précédentes, dans lequel les billes (6) sont revêtues au moins en partie de la substance réfléchissante.

11. Repère (1) selon l'une des revendications précédentes, dans lequel le repère (1) présente une forme sphérique, cylindrique ou pyramidale ou la forme d'un prisme.

12. Repère (1) selon l'une des revendications précédentes, dans lequel le repère (1) présente un diamètre compris entre 5 et 50 mm.
